# NOUVEAU FASCICULE DE BREVET EUROPEEN

(11) **EP 0 665 861 B2**
(45) Date de publication et mention de la décision concernant l'opposition: **23.06.2004**
(45) Mention de la délivrance du brevet: 01.07.1998
(21) Numéro de dépôt: 93923586.7
(22) Date de dépôt: 19.10.1993
(51) Int. Cl.: C08J 3/03, C08J 3/05, C08L 83/04

(54) **PROCEDE DE PREPARATION D'EMULSIONS AQUEUSES D'HUILES ET/OU DE GOMMES ET/OU DE RESINES SILICONES**
VERFAHREN ZUR HERSTELLUNG VON WÄSSRIGEN ÖL- UND/ODER GUMMI- UND/ODER SILIKONHARZ-EMULSIONEN
METHOD FOR PREPARING OIL-IN-WATER EMULSIONS OF OILS AND/OR GUMS AND/OR SILICONE RESINS

(30) Priorité: 20.10.1992 FR 9212519
(43) Date de publication de la demande: 09.08.1995
(73) Titulaire: RHODIA CHIMIE, 92408 Courbevoie Cédex (FR)
(72) Inventeur: DERIAN, Paul-Jo[l, F-92260 Fontenay-aux-Roses (FR); FEDER, Michel, F-69003 Lyon (FR); PAILLET, Jean-Pierre, F-69320 Feyzin (FR); PEIGNIER, Michel, F-69210 Lentilly (FR); SENECHAL, Alain, F-94700 Maison-Alfort (FR); ULRICH, Jean, F-69360 Ternay (FR)
(74) Mandataire: Trolliet, Maurice
(86) Numéro de dépôt international: PCT/FR1993/001026
(87) Numéro de publication internationale: WO 1994/009058

(56) Documents cités:
- EP-A- 0 200 916
- EP-A- 0 270 898
- EP-A- 0 463 431
- DE-A- 1 644 952
- DE-A- 3 932 025
- GB-A- 1 191 289
- Chem.-Ing.-Tech. 61(9), 701-711 (1989)
- Römpp, 9.Aufl. 1989

## Description

La présente invention a pour objet un procédé de préparation d' émulsions d'huiles et/ou de gommes et/ou de résines silicones de préférence visqueuses.

Il a été proposé (brevet anglais 1,191,289) d'émulsifier des huiles silicones visqueuses (5 000 -1 000 000 mPa.s.) par malaxage d'un mélange d'huile, de 1-10% d'agent tensio-actif et de 10-20 % d'eau, à l'aide d'un broyeur à cylindres (roll-mill mixer) équipé d'au moins deux rouleaux ; un tel procédé présente l'inconvénient de mettre en oeuvre un appareillage de faible productivité présentant en outre des problèmes de sécurité, ce qui le rend difficilement exploitable industriellement.

La demande européenne EP -A- 463 431 décrit la préparation d'émulsions aqueuses d'huiles silicones, notamment de haute viscosité, par introduction séparée en deux étapes de deux types d'agents tensio-actifs de HLB différents dans un appareil classique de malaxage.

La demanderesse a trouvé un procédé de préparation d'émulsions aqueuses de phases visqueuses à base d'huiles et/ou de gommes et/ou de résines silicones de préférence visqueuses, utilisant un appareil classique de malaxage, et ne nécessitant pas l'emploi obligatoire de deux types d'agents tensio-actifs.

Un tel procédé permet de préparer des émulsions d'huiles et/ou de gommes et/ou de résines silicones de granulométrie parfaitement maîtrisée et relativement homogène.

Le procédé de préparation d'émulsions d'huiles et/ou de gommes et/ou de résines silicones faisant l'objet de l'invention, est caractérisé :
- en ce qu'on malaxe un mélange constitué de
   . 100 parties en poids d'une phase silicone (A) de viscosité dynamique, mesurée à 25 °C à l'aide d'un viscosimètre Brookfield selon les indications de la norme AFNOR NFT 76 102 de février 1972, au moins égale à 3 Pa.s., ou de consistance déterminée par mesure à 25 °C de la pénétrabilité à l'aide d'un pénétromètre selon les indications de la norme AFNOR NFT 60 123 ou NFT 66 004, inférieure à 2000, phase silicone comprenant au moins une huile et/ou au moins une gomme et/ou au moins une résine polyorganosiloxanique ;
   . 2 à 20 parties en poids d'eau ;
   . 3 à 20 parties en poids d'au moins un agent tensio-actif (B) de nature non-ionique ou une combinaison de 0,5 à 10 parties en poids d'au moins un agent tensio-actif (B) de nature non-ionique et de 2,5x10⁻⁴ à 20 parties en poids d'au moins un polymère hydrosoluble épaississant (C) de masse moléculaire supérieure à 10 000 g./mole,
- ledit agent tensio-actif ou mélange d'agents tensio-actifs présentant un HLB d'au moins 10 et les quantités relatives d'eau, de(s) constituant(s) (B) et éventuellement (C) étant telles que la viscosité ou la consistance du mélange eau + agent(s) tensio-actif(s) + polymère(s) hydrosoluble(s) épaississant(s) éventuel(s), ladite viscosité ou consistance étant mesurée selon la méthode normalisée indiquée ci-avant, soit voisine du ou supérieure au dixième de la viscosité ou consistance de la phase silicone (A)
- ledit malaxage étant réalisé, en mettant en oeuvre une seule étape, dans un malaxeur muni d'un agitateur, agitateur dont la partie mobile ne tourne pas à plus de 2500 tours/min, avec une vitesse tangentielle en extrêmité de partie mobile ne dépassant pas 20 m/s et avec un rapport vitesse tangentielle en extrêmité de partie mobile/distance entre l'extrêmité de la partie mobile et la paroi du malaxeur qui est inférieur à 50000 s⁻¹, pendant une durée suffisante pour obtenir une émulsion de type "huile dans eau" de granulométrie de l'ordre de 0,1 à 5 micromètres ;
- puis en ce qu'on dilue éventuellement le milieu avec de l'eau en fonction du taux d'extrait sec désiré.

De préférence, le procédé selon la présente invention est caractérisé :
- en ce qu'on malaxe un mélange constitué de
   . 100 parties en poids d'une phase silicone (A) de viscosité dynamique à 25 °C au moins égale à 30 Pa.s., ou de consistance à 25 °Cinférieure à 2000, phase silicone comprenant au moins une huile et/ou au moins une gomme et/ou au moins une résine polyorganosiloxanique ;
   . 3 à 15 parties en poids d'eau ;
   . 5 à 15 parties en poids d'au moins un agent tensio-actif (B) de nature non-ionique ou une combinaison de 1 à 10 parties en poids d'au moins un agent tensio-actif (B) de nature non-ionique et de 0,001 à 15 parties en poids d'au moins un polymère hydrosoluble épaississant (C) de masse moléculaire supérieure à 100 000 g./mole,
- ledit agent tensio-actif ou mélange d'agents tensio-actifs présentant un HLB d'au moins 10 et les quantités relatives d'eau, de(s) constituant(s) (B) et éventuellement (C) étant telles que la viscosité ou la consistance du mélange eau + agent(s) tensio-actif(s) + polymère(s) hydrosoluble(s) épaississant(s) éventuel(s) soit voisine de ou supérieure à la viscosité ou consistance de la phase silicone (A) ;
- ledit malaxage étant réalisé, en mettant en oeuvre une seule étape, dans un malaxeur muni d'un agitateur, agitateur dont la partie mobile ne tourne pas à plus de 2500 tours/min, avec une vitesse tangentielle en extrêmité de partie mobile ne dépassant pas 20 m/s et avec un rapport vitesse tangentielle en extrêmité de partie mobile / distance entre l'extrêmité de la partie mobile et la paroi du malaxeur qui est inférieur à 50000 s⁻¹, pendant une durée suffisante pour obtenir une émulsion de type "huile dans eau" de granulométrie de l'ordre de 0,2 à 3 micromètres ;
- puis en ce qu'on dilue éventuellement le milieu avec de l'eau en fonction du taux d'extrait sec désiré.

Comme exemples de phases silicones (A) pouvant être mises en oeuvre, on peut citer celles consistant en :
. une huile et/ou une gomme et/ou une résine polyorganosiloxanique de viscosité au moins égale à 3 Pa.s., de préférence de 'ordre de 30 à 2.500 Pa.s. ou de consistance de l'ordre de 200 à 2.000.
. un mélange d'huile(s) et/ou de gomme(s) et/ou de résine(s) polyorganosiloxanique(s), mélange de viscosité au moins égale à 3 Pa.s., de préférence de l'ordre de 30 à 2.500 Pa.s. ou de consistance de l'ordre de 200 à 2.000 ;
. un mélange d'huile(s) et/ou gomme(s) et/ou résine(s) polyorganosiloxanique(s) et d'au moins un solvant de ladite huile et/ou gomme et/ou résine et/ou d'au moins un silane et/ou d'au moins une charge siliceuse ou non siliceuse, mélange de viscosité au moins égale à 3 Pa.s., de préférence de l'ordre de 30 à 2.500 Pa.s. ou de consistance de l'ordre de 200 à 2.000.

Parmi les huiles et les gommes polyorganosiloxaniques pouvant être mises en oeuvre, on peut citer celles constituées des motifs de formule

R'₃₋ₐ Rₐ Si O_{1/2} et R₂ Si O

formules où
- a est un entier de 0 à 3
- les radicaux R sont identiques ou différents et représentent
   . un groupe hydrocarboné aliphatique saturé ou insaturé contenant de 1 à 10 atomes de carbone ;
   . un groupe hydrocarboné aromatique contenant de 6 à 13 atomes de carbone ;
   . un groupe organique polaire lié au silicium par un liaison Si-C ou Si-O-C ;
   . un atome d'hydrogène ;
- les radicaux R' sont identiques ou différents et représentent
   . un groupe OH
   . un groupe alkoxy ou alcényloxy contenant de 1 à 10 atomes de carbone ;
   . un groupe aryloxy contenant de 6 à 13 atomes de carbone ;
   . un groupe acyloxy contenant de 1 à 13 atomes de carbone
   . un groupe cétiminoxy contenant de 1 à 8 atomes de carbone
   . un groupe amino- ou amido-fonctionnel contenant de 1 à 6 atomes de carbone, lié au silicium par une liaison Si-N
de préférence au moins 80 % des radicaux R desdites huiles représentant un groupe méthyle.

Parmi les résines polyorganosiloxanes pouvant être mises en oeuvre, on peut citer celles constituées de motifs de formules

R Si O _{3/2} (motif T) et/ou Si O₂ (motif Q)

associés à des motifs de formule

R'₃₋ₐ Rₐ Si O_{1/2} (motif M) et/ou R₂ Si O (motif D)

formules dans lesquelles a, R et R' ont la définition donnée ci-dessus.

Celles-ci sont généralement du type MQ, MDQ, TDM, TD, MT.

A titre d'exemples de radicaux hydrocarbonés aliphatiques ou aromatiques R, on peut citer les groupes :
. alkyles tels que par exemple méthyle, éthyle, octyle, trifluoropropyle;
. alkoxyalkylène tels que par exemple -CH₂-CH₂-O-CH₃ ; -CH₂-CH₂-O-CH₃ ;
. alcényles tels que par exemple vinyle, allyle, hexényle, décényle, décadiényle;
. alcényloxyalkylène tels que -(CH₂)₃-O-CH₂-CH=CH₂ ;

   - (CH₂)₃-OCH₂-CH₂-O-CH=CH₂ ;
. aryles tels que par exemple phényle.

A titre d'exemples de groupes organiques polaires R, on peut citer les groupes :
. hydroxyfonctionnels tels que par exemple -(CH₂)₃-OH ; -(CH₂)₄N(CH₂CH₂OH)₂; - (CH₂)₃-N(CH₂CH₂OH)-CH₂-CH₂-N(CH₂CH₂OH)₂;
. aminofonctionnels tels que par exemple -(CH₂)₃-NH₂ ; -(CH2)₃-NH-(CH₂)₂NH₂ ;
. amidofonctionnels tels que par exemple -(CH₂)₃-N-(COCH₃)-(CH₂)₂NH(COCH₃);
. carboxyfonctionnels tels que par exemple -CH₂-CH₂-S-CH₂-COOH.

A titre d'exemples de radicaux R', on peut citer les groupes :
. alkoxy tels que par exemple méthoxy, éthoxy, octyloxy ;
. alcenyloxy tels que par exemple vinyloxy, héxényloxy, isopropényloxy ;
. aryloxy tels que par exemple phényloxy ;
. acyloxy tels que par exemple acétoxy ;
. cétiminoxy tels que par exemple ON=C(CH₃)C₂H₅ ;
. aminofonctionnels tels que par exemple éthylamino, phénylamino;
. amidofonctionnel tels que méthylacétamido.

A titre d'exemples concrets de "motifs D" on peut citer :
(CH₃)₂SiO ; CH₃(CH=CH₂)SiO ; CH₃(C₆H₅)SiO ; (C₆H₅)₂SiO ; CH₃HSiO ; CH₃(CH₂-CH₂-CH₂OH)SiO ;

A titre d'exemples concrets de "motifs M", on peut citer:
(CH₃)₃SiO_{1/2} ; (CH₃)₂(OH)SiO_{1/2} ; (CH₃)₂(CH=CH₂)SiO_{1/2} ; (CH₃)₂HSiO_{1/2} ; (OCH₃)₃SiO_{1/2} ; [O-C(CH₃)=CH₂]₃SiO_{1/2} ; [ON=C(CH₃)]₃SiO_{1/2} ; (NH-CH₃)₃ SiO_{1/2} ; (NH-CO-CH₃)₃SiO_{1/2}

A titre d'exemples concrets de "motifs T", on peut citer
CH₃SiO_{3/2} ; (CH=CH₂)SiO_{3/2} ; HSiO_{3/2.}

Lorsque lesdites huiles, gommes ou résines contiennent des radicaux R réactifs et/ou polaires (tels que par exemple H, OH, vinyle, allyle, héxényle, aminoalkyles), ces derniers ne représentent généralement pas plus de 2 % du poids de l'huile ou de gomme et pas plus de 10 % du poids de la résine.

Les huiles polydiméthylsiloxanes et α,ω-bis(hydroxy)polydiméthylsiloxanes visqueuses ainsi que les gommes polydiméthylsiloxanes, polyphénylméthylsiloxane et α,ω-bis(hydroxy)polydiméthylsiloxanes sont des produits du commerce bien connus.

Les résines visqueuses polyméthylsiloxanes DT contenant de 1 à 2 % en poids de fonctions silanols sont également des produits du commerce.

Parmi les solvants des huiles, gommes ou résines silicones, éventuellement présents dans la phase silicone, on peut citer les organopolysiloxanes cycliques volatils (par exemple octaméthylcyclotétrasiloxane, decaméthylcyclopentasiloxane), les huiles polydiméthylsiloxanes courtes (viscosité inférieure à 100 mPa.s.), l'hexaméthyldisiloxane, les cétones (par exemple méthyléthylcétone)les éthers (par exemple éther diéthylique), les esters (par exemple myristate d'isopropyle, acétate d'éthyle), certains solvants chlorés ou chlorofluorés (par exemple chlorure de méthylène, chloroforme), les paraffines très ramifiées (par exemple huiles blanches à base d'isoalcanes et de cycloalcanes)

Selon la présente invention, peuvent en outre être présents dans la phase silicone à émulsifier, des silanes et/ ou des charges minérales diverses.

Ces silanes peuvent être notamment des sous-produits de synthèse desdites huiles, gommes ou résines polyorganosiloxaniques mises en oeuvre ou des agents de réticulation desdites huiles, gommes ou résines.

Ils peuvent être représentés par la formule (R_{b}) Si (OR')_{4-b} , formule dans laquelle b est un entier de 0 à 4, R et R' ayant la définition donnée ci-dessus.
Ils sont notamment décrits dans US-A-3 294 725 ; US-A- 4 584 341 ; US-A- 4 618 642 ; US-A-4 608 412 ; US-A-4 525 565 ; EP-A-340 120 ; EP-A-364 375 ; FR -A-1 248 826 ; FR-A-1 423 477 ; EP-A-387 157.

A titre d'exemples, on peut citer les silanes suivants :
Si(OC₂H₅)₄ ; CH₃Si(OCH₃)₃ ; CH₃Si(OC₂H₅)₃ ; (C₂H₅O)₃Si(OCH₃) ;
CH₂=CHSi(OCH₃)₃ ; CH₃(CH₂=CH)Si(OCH₃)₂ ; CH₂=CHSi(OC₂H₅)₃ ;
CH₂=CHSi[ON=C(CH₃)C₂H₅]₃ ; CH₃Si[ON=C(CH₃)₂]₃ ;
CH3Si[O-C(CH3)=CH2]3 ; méthyltris(Nméthylacétamidosilane) , méthyltris(cyclohéxylaminosilane).

Ils sont généralement présents selon des quantités de l'ordre de 0 à 10 parties en poids, de préférence de l'ordre de 0 à 5 parties en poids pour 100 parties en poids d'huile(s) et/ou de gomme(s) et/ou de résine polyorganosiloxanique lorsqu'il s'agit de sous-produits réactionnels.

Lorsque leur fonction d'agent de réticulation des huiles, gommes ou résines hydroxylées est recherchée, ils sont généralement présents selon des quantités de l'ordre de 0,5 à 30 parties en poids, de préférence de l'ordre de 2 à 8 parties en poids pour 100 parties en poids d'huile(s) et/ ou de gomme(s) et/ou de résine(s) .

Lesdits silanes peuvent aussi être un additif permettant de moduler les propriétés physicochimiques, d'adhérence notamment des compositions silicones d'applications diverses obtenues à partir des émulsions aqueuses préparées selon le procédé de l'invention. Des exemples de tels silanes sont notamment décrits dans EP-A- 340 120. Parmi cette catégorie de silanes on peut citer par exemple l'aminopropyltriéthoxysilane, l'aminopropylméthyldiéthoxysilane ; le glycidoxypropyltriméthoxysilane.

Ils sont mis en oeuvre selon des quantités pouvant aller jusqu'à 200 %, généralement de l'ordre de 2 à 100 % du poids d'huile(s) et/ou de gomme(s) et/ ou résine(s).

Selon l'invention peuvent être présentes des charges siliceuses ou non siliceuses renforçantes ou semi-renforçantes ; à titre d'exemple, on peut citer les silices colloïdales, les poudres de silice de combustion et de précipitation, les terres de diatomées, le quartz broyé, le carbonate de calcium naturel, l'alumine hydratée, l'hydroxyde de magnésium, le noir de carbone, le dioxyde de titane, l'oxyde d'aluminium, la vermiculite, l'oxyde de zinc, le mica, le talc, l'oxyde de fer, le sulfate de baryum, la chaux éteinte ; la granulométrie de ces charges est généralement de l'ordre de 0,001 à 300 µm. ; elles sont généralement présentes selon des quantités pouvant aller jusqu'à 300 %, de préférence de l'ordre de 3 à 100 % du poids d'huile(s) et/ou de gomme(s) et/ou de résine(s)

Les agents tensio-actifs (B) mis en oeuvre sont de nature non-ionique, de HLB d'au moins 10, de préférence de l'ordre de 10 à 20.

Les agents tensio-actifs non-ioniques sont choisis parmi, par exemple, les acides gras alcoxylés, les alkylphénols polyalcoxylés, les alcools gras polyalcoxylés, les amides gras polyalcoxylés ou polyglycérolés, les alcools et les alphadiols polyglycérolés, les polymères blocs oxyde d'éthylène-oxyde de propylène, ainsi que les alkylglucosides, les alkylpolyglucosides, les sucroéthers, les sucroesters, les sucroglycérides, les esters de sorbitan, et les composés éthoxylés de ces dérivés de sucres présentant un HLB d'au moins 10.

Le (mélange de) tensio-actif(s) est choisi en fonction de la nature de l'huile et/ou de la gomme et/ou de la résine polyorganosiloxane; un HLB de l'ordre de 11 à 15 est généralement choisi pour émulsifier une huile ou une gomme silicone (A) constituée par un α,ω-bis(triméthyl)polydiméthylsiloxane ou un α,ω -bis(hydroxy)polydiméthylsiloxane.

Les polymères épaisissants (C) sont solubles à au moins 50 % dans l'eau ; à titre d'exemples de polymères épaissants, on peut citer
. ceux obtenus par synthèse chimique, tels que par exemple les alcools polyvinyliques, les polyéthylènes glycols, les polyvinylpyrrolidones, les polyacrylates de métaux alcalins,
. ceux extraits de végétaux et éventuellement modifiés, tels que par exemple les carraghénanes, les alginates, les carboxyméthylcelluloses, les méthylcelluloses, les hydroxypropylcelluloses, les hydroxyéthylcelluloses,
. ceux obtenus par biosynthèse tels que par exemple la gomme xanthane.

Les quantités relatives d'eau, d'agent(s) tensio-actif(s) et de polymère(s) épaississant(s) éventuel(s) sont fonctions de la viscosité de la phase silicone comprenant au moins une huile et/ou une gomme et/ou une résine polyorganosiloxane ainsi que de la nature du (mélange de) tensio-actif(s) et de la nature du (mélange de) polymère(s) épaississant(s) éventuel(s).

En l'absence de polymère épaississant, le rapport pondéral eau / eau+agent(s) tensio-actif(s) est par exemple de l'ordre de 20/100 à 70/100, préférentiellement de l'ordre 25/100 à 60/100 pour stabiliser une émulsion d'une phase silicone consistant en une huile α,ω-bis(triméthyl) ou (hydroxy) polydiméthylsiloxane de viscosité de l'ordre de 30 Pa.s.à 500 Pa.s., à l'aide d'un nonylphénol présentant 9 ou 10 motifs éthoxy comme seul agent tensio-actif.

L'opération de mise en émulsion de la phase silicone peut être réalisée par
. introduction d' au moins une huile et/ou une gomme et/ou au moins une résine +solvant(s) éventuel(s)+ silane(s) éventuel(s) dans un mélange eau + agent(s) tensio-actif(s)+polymère(s) hydrosoluble(s) éventuel(s), la ou les charge(s) eventuelle(s) pouvant être présente(s) dans le mélange aqueux et/ou introduite(s) dans ledit mélange, puis malaxage à une température de l'ordre de 10 à 50 °C;
. ou de préférence introduction de l'eau dans un mélange huile(s) et/ou gomme(s) et/ou résine(s) présente(s) en totalité ou partiellement [par exemple 50-90 % de la quantité totale d'huile(s) et/ou gomme(s) et/ou résine(s)]+ solvant(s) éventuel(s)+silane(s) éventuel(s) + charge(s) éventuelle(s)+ agent(s) tensio-actif(s) +polymère(s) hydrosoluble(s) éventuel(s), puis malaxage à une température de l'ordre de 10 à 50 °C, la quantité éventuellement restante d'huile(s) et/ou gomme(s) et/ou résine(s) étant introduite dans le milieu après la formation de l'émulsion "huile dans eau" tout en maintenant le malaxage.

Tout appareil classique de malaxage peut être mis en oeuvre, notamment les appareils à agitation lente. Comme indiqué supra, l'opération de malaxage est réalisée dans un malaxeur muni d'un agitateur, agitateur dont la partie mobile ne tourne pas à plus de 2500 tours/min (de préférence à pas plus de 1500 tours/min et tout particulièrement à pas plus de 500 tours/min) avec une vitesse tangentielle en extrémité de partie mobile ne dépassant pas 20m/s (de preférence ne dépassant pas 5m/s et tout particulièrement ne dépassant pas 2,5m/s) et avec un rapport vitesse tangentielle en extrémité de partie mobile / distance entre l'extrémité de la partie mobile et la paroi du malaxeur qui est inférieur à 50000s⁻¹ (de préférence inférieur à 10000s⁻¹ et tout particulièrement inférieur à 2500s⁻¹).

A titre d'exemple on peut citer les extrudeuses à vis simple ou multiple(s), les malaxeurs planétaires, les malaxeurs à crochet, les disperseurs lents, les malaxeurs statiques, les malaxeurs à pale, à hélice, à bras, à ancre.

Les émulsions d'huiles et/ou de gommes et/ou résines silicones visqueuses obtenues selon le procédé de l'invention sont particulièrement stables au stockage ; elles peuvent être très fines et monodispersées ; leur taux d'extrait sec peut aller de 25 (ou moins selon l'application visée desdites émulsions) à 98 %, le réglagle de l'extrait sec étant réalisé, au besoin, par dilution avec de l'eau.

Elles peuvent être utilisées pour la préparation de compositions à base de silicones adhérant sur des surfaces en matériaux divers tels que verre, béton, bois, pour la préparation de produits cosmétiques (shampoings, crèmes, agents conditionneurs, savons liquides ou autres produits de l'hygiène corporelle), de produits de nettoyage industriel ou ménager, de produits détartrants ménagers, de produits de polissage de surfaces (par exemple pour voiture), de produits cirants (par exemple pour chaussures), pour le traitement adoucissant des textiles.

Les exemples sont donnés à titre indicatif et ne peuvent être considérés comme une limite du domaine et de l'esprit de l'invention.

### Exemple 1

On introduit dans un malaxeur à bras de type KUSTNER® (commercialisé par Kustner) de 5 litres
. 1.500 g d'huile α,ω-bis(hydroxy)polydimethylsiloxane de viscosité égale à 175 Pa.s. (huile 48 V 175000)
. puis 75 g de CEMULSOL NP 9® (nonylphénol contenant 9 motifs ethoxy commercialisé par Rhône-Poulenc) présentant un HLB de 12,8

Le milieu est agité pendant 15 minutes environ à 90 tours / minute .

On coule alors lentement 100 g d'eau en 10 minutes environ sous la même agitation, puis on malaxe le milieu sous la même agitation pendant 150 minutes environ ; le rapport pondéral eau / eau+tensio-actif est de 0,57.
(la viscosité dynamique de ce mélange 75/100 tensio-actif/eau est de 380 Pa.s. à un gradient de cisaillement de 1 s·⁻¹)

On suit, à l'aide d'un granulomètre COULTER N4S® (commercialisé par COULTRONICS), l'évolution de la granulométrie moyenne de l'émulsion formée en fonction du temps de malaxage.

L'émulsion finale est ensuite diluée avec 584 g d'eau afin d'obtenir un extrait sec de 70 %

### Exemple 2

On répète l'opération décrite à l'exemple 1 en mettant en oeuvre 50 g d'eau introduits en 5 minutes environ, au lieu de 100 g, ce qui correspond à un rapport eau/eau+ tensio-actif de 0,4.

On constate d'après le tableau 1 suivant que l'émulsion devient fine plus rapidement.

**TABLEAU 1**

| | **granulométrie moyenne** (en nanomètres) | |
|---|---|---|
| **durée de malaxage** après introduction de l'eau (en minutes) | **exemple 1** | **exemple2** |
| 30 | 1700 | 473 |
| 60 | 1490 | 413 |
| 90 | 567 | 373 |
| 120 | 437 | - |
| 150 | 375 | 340 |

### Exemple 3

Dans un réacteur de 250 cm³, muni d'un système d'agitation à ancre, on introduit
. 2,5g. de SIPONIC L4 ® commercialisé par RHONE-POULENC
. 2,5g. d'eau

Le mélange est agité à 150 tours/min. pendant 5 minutes.

Il se forme une pâte épaisse à laquelle on ajoute en 20 minutes environ, sous agitation à environ 350 tours/min., 87 g. d'huile polydiphényldiméthylsiloxane 70641V500 000 (viscosité de 500 000mPa.s.). A la fin de l'introduction de l'huile, le mélange est malaxé sous la même agitation pendant 15 minutes environ. On obtient une émulsion présentant un extrait sec de 70% et une granulométrie de 2,76µm..

### Exemple 4

On répète l'opération décrite à l'exemple 1 à partir de :
. 1500 g d'huile α,ω-bis(hydroxy)polydiméthylsiloxane de 70 Pa. s. de viscosité
. 75 g de Cemulsol NP 9
. 75 g d'eau introduits en 5 min environ, ce qui correspond à un rapport eau / eau+tensio-actif de 0,5.

Les granulométries moyennes de l'émulsion obtenue après 30, 60, 90, minutes de malaxage à 90 tours/minute sont respectivement de 600, 487 et 495 nanomètres. L'émulsion finale est diluée avec 568 g d'eau pour obtenir un extrait sec de 71 %.

### Exemple 5

On répète l'opération décrite à l'exemple 1 à partir de
. 1500 g d'huile 48 V 175000
. 150 g de Cemulsol NP9
. 150 g d'eau introduites en 10 min environ
   On obtient après 90 minutes de malaxage à 90 tours / minute une émulsion stable de 380 nm.
   Celle-ci est diluée à 75 % d'extrait sec par ajout de 555 g d'eau et remalaxage pendant 20 minutes.

### Exemple 6

On introduit dans le malaxeur de l'exemple 1
. 1500 g d'huile 48 V 175000
. 37,5 g de CEMULSOL NP 5® (nonylphénol à 5 motifs ethoxy commercialisé par Rhône-Poulenc)
. 112,5 g de CEMULSOL NP 12® (nonylphénol à 12 motifs ethoxy commercialisé par Rhône-Poulenc)

Le mélange de tensio-actifs présente un HLB de 13. Après malaxage pendant 20 minutes à 90 tours/minute, on rajoute 100 g d'eau en 6 min environ ; on malaxe dans les mêmes conditions pendant 1 heure ; on rajoute 30 g d'eau supplémentaires en 2 min environ et on agite dans les mêmes conditions pendant 1 heure. La granulométrie moyenne de l'émulsion obtenue est de 580 nm.

L'émulsion est amenée à un extrait sec de 75 % par dilution avec 470 g d'eau.

### Exemple 7

Dans le malaxeur de l'exemple 1, on introduit
. 1500 g d'huile 48 V 175000
. un mélange préalablement chauffé à 50 °C de
. 45 g de GENAPOL X 050® (alcool gras éthoxylé commercialisé par Hoechst)
. 45 g de GENAPOL UD 110® (alcool gras éthoxylé commercialisé par Hoechst)

Le mélange de tensio-actif présente un HLB de 12,5.

Après malaxage pendant 20 minutes à 90 tours/minute, on introduit 105 g d'eau en 6 min environ.

On obtient par malaxage à 90 tours/minute un émulsion bipopulée ; l'évolution de la granulométrie moyenne de l'émulsion en fonction du temps de malaxage figure au tableau 2.

L'extrait sec de l'émulsion finale est ajusté à 76 % par addition de 440 g d'eau et remalaxage à 90 tours/minute pendant 20 minutes.

**TABLEAU 2**

| | | | | |
|---|---|---|---|---|
| **durée de malaxage** (minutes) | 30 | 60 | 90 | 120 |
| 1ère population ⌀ **moyen** (nm.) | 1530 | 1500 | 949 | 785 |
| proportion (%) | 78 | 83 | 89 | 95 |
| 2ème population ⌀ **moyen** (nm.) | 573 | 433 | 303 | 298 |
| proportion (%) | 22 | 17 | 11 | 5 |

### Exemple 8

### Préparation d'une huile méthoxyfonctionnelle visqueuse

On introduit dans le malaxeur de l'éxemple 1
. 1500 g d'huile 48 V 175000
. 15 g de vinyltrimethoxysilane

Après 10 minutes d'agitation à 90 tours/minute, on ajoute 7,95 g d'une solution méthanolique à 3,75 % de lithine (LiOH,H₂O) ; on agite pendant 25 minutes dans les mêmes conditions avant de neutraliser le mélange par ajout de 6 g du produit de réaction de l'acide phosphorique avec l'octaméthylcyclotétrasiloxane titrant 8,5 % de H₃ PO₄.

### Emulsification de l'huile méthoxyfonctionnelle

On ajoute à l'huile obtenue un mélange de
. 75 g de Cemulsol NP 5
. et de 75 g de Cemulsol NP 12

Le mélange de tensio-actifs présente un HLB de 12.

Le milieu est malaxé à 90 tours/minute pendant 10 minutes ; on ajoute lentement 130 g d'eau en 8 min environ, puis on malaxe pendant 90 minutes à 90 tours/minute. On obtient une émulsion de 535 nm. de granulométrie moyenne, émulsion que l'on dilue avec 420 g d'eau.

### Exemple 9

### Préparation d'une huile méthoxyfonctionnelle visqueuse

On répète l'opération décrite à l'exemple 8 en mettant en oeuvre 45 g de vinyltriméthoxysilane au lieu de 15 g .

### Emulsification de l'huile méthoxyfonctionnelle

On ajoute à l'huile obtenue, 150 g de CEMULSOL NP 7® (nonylphénol à 7 motifs éthoxy commercialisé par Rhône-Poulenc) de HLB = 11,7.

On malaxe pendant 5 minutes à 90 tours/minutes, puis on ajoute 160 g d'eau en 10 min environ et malaxe pendant 90 minutes à 90 tours/minute.

L'émulsion obtenue a une granulométrie moyenne de 1670 nm. ; elle est ensuite diluée par addition de 390 g d'eau.

### Exemple 10

On introduit dans le malaxeur de l'exemple 1
. 1525 g d'huile 48 V 175000
. 15 g de N-méthyl-3-aminopropyl-trimethoxysilane

Le mélange est malaxé pendant 20 minutes à 90 tours/minute, puis mis sous vide léger pendant 5 minutes.

On ajoute un prémélange de
. 70 g de Cemulsol NP 5 et de
. 70 g de Cemulsol NP 9

Le mélange de tensio-actifs présente un HLB de 11,4 .

On malaxe pendant 10 minutes à 90 tours/minute, puis on introduit 120 g d'eau en 7 mn environ sous agitation ; le mélange est malaxé pendant 45 minutes à 90 tours/minute.

L'émulsion obtenue présente une granulométrie moyenne de 2400 nm.

On rajoute 30 g d'eau en 3mn environ et on continue à malaxer dans les mêmes conditions pendant 90 minutes ; la granulométrie est alors de 1790 nm.

L'émulsion est diluée avec 400 g d'eau pour avoir un extrait sec de 74 %.

### Exemple 11

### Préparation d'une huile méthoxyfonctionnelle

On introduit dans le malaxeur de l'exemple 1
. 1500 g d'huile 48 V 175000
. 45 g de vinyltriméthoxysilane

Après 5 minutes d'agitation à 90 tours/min , on ajoute 12 g d'une solution méthanolique à 3,75 % de lithine ; on agite pendant 20 minutes à 90 tours/min, puis on neutralise le mélange par 9 g du produit de réaction de l'acide phosphorique avec l'octaméthyltétracyclosiloxane titrant à 8,5 % de H₃PO₄.

Après 15 minutes d'agitation dans les mêmes conditions, on désaère sous vide pendant 15 minutes.

### Emulsification de l'huile méthoxyfonctionnelle

On ajoute à l'huile ainsi obtenue un mélange de
. 75 g de Cemulsol NP 5 et de
. 75 g de Cemulsol NP 12

On agite pendant 5 minutes à 90 tours/min, puis on introduit 130 g d'eau en 8 min environ.

Après 90 minutes de malaxage à 90 tours/minute, on obtient une émulsion de granulométrie moyenne de 630 nm.

Elle est ensuite diluée par ajout de 420 g d'eau.

### Exemple 12

Dans un réacteur de 250 cm³, muni d'un système d'agitation à pale raclante, on introduit
. 5,6 g. de SOPROPHOR NP10® (nonylphénol éthoxylé à 10 motifs oxyde d'éthylène commercialisé par RHONE-POULENC) de HLB 13,3
. 3 g. de silice hydrophile AEROSIL 200® (commercialisée par DEGUSSA) dont la granulométrie est de 0,012 micromètre
. 8,4 g. d'eau

Le mélange est agité à 150 tours/min. pendant 5 minutes.

Il se forme une pâte épaisse à laquelle on ajoute en 20 minutes environ, sous agitation à environ 350 tours/min., 83 g. d'huile α,ω-bis(hydroxy)polydiméthylsiloxane de viscosité égale à 70 Pa.s. (huile 48V70 000). A la fin de l'introduction de l'huile, le mélange est malaxé sous la même agitation pendant 45 minutes environ. On obtient une émulsion présentant un extrait sec de 91,6 % et un granulométrie de 1,14 micromètre.

### Exemple 13

On répète l'opération décrite à l'exemple 12 en mettant en oeuvre
- d'une part,
   . 5 g. de SOPROPHOR NP10
   . 6 g. de silice AEROSIL 200® (commercialisée par DEGUSSA)
   . 9 g. d'eau
- et d'autre part, 80 g. d'huile 48V70 000

L'émulsion obtenue présente un extrait sec de 91 % et une granulométrie de 1,06 micromètre.

### Exemple 14

On répète l'opération décrite à l'exemple 12, en mettant en oeuvre
- d'une part,
   . 4,9 g. de SOPROPHOR NP10
   . 6 g. de silice hydrophile TIXOSIL 375® (commercialisé par RHONE-POULENC) de granulométrie de 1,6 micromètre
   . 9 g. d'eau
- et d'autre part, 81 g. d'huile 48V70 000

L'émulsion obtenue présente un extrait sec de 91,9 % et une granulométrie de 0,99 micromètre.

### Exemple 15

Dans un réacteur de 250 cm³, muni d'un système d'agitation à pale raclante, on introduit
. 5 g. de SOPROPHOR NP10®
. 7 g. d'eau

Le mélange est agité à 150 tours/min. pendant 5 minutes.

Il se forme une pâte épaisse à laquelle on ajoute en 20 minutes environ, sous agitation à environ 350 tours/min., 85 g. d'huile 48V70 000 et 3 g. de silice hydrophile TIXOSIL 375. A la fin de l'introduction de l'huile et de la silice, le mélange est malaxé sous la même agitation pendant 45 minutes environ. On obtient une émulsion présentant un extrait sec de 93 % et un granulométrie de 1,16 micromètre.

### Exemple 16

Dans un réacteur de 250 cm³, muni d'un système d'agitation à pale raclante, on introduit
. 6,3 g. de SOPROPHOR NP10®
. 7,7 g. d'eau
(la viscosité dynamique à un gradient de cisaillement de 1 s⁻¹ de ce mélange tensio-actif/eau est de l'ordre de 300 Pa.s.)

Le mélange est agité à 150 tours/min. pendant 5 minutes.

Il se forme une pâte épaisse à laquelle on ajoute en 20 minutes environ, sous agitation à environ 350 tours/mn., 86 g. d'huile 48V70 000 . A la fin de l'introduction de l'huile, le mélange est malaxé sous la même agitation pendant 75 minutes environ. On obtient une émulsion présentant un extrait sec de 92,3 %.

L'évolution de la granulométrie moyenne en fonction du temps de malaxage est donnée au tableau 3.

### Exemple 17

On répète l'opération décrite à l'exemple 16 à partir
- d'une part
   . 6,3 g. de CEMULSOL NP10® de HLB 13,3
   . 7,7 g. d'eau
- et d'autre part 86 g. d'huile α,ω-bis(triméthyl)polydiméthylsiloxane ayant une viscosité de 100 Pa.s. (huile 47V100 000)

L'émulsion obtenue présente un extrait sec de 92,3 %.

L'évolution de la granulométrie moyenne en fonction du temps de malaxage est donnée au tableau 3.

### Exemple 18

On répète l'opération décrite à l'exemple 16 à partir
- d'une part
   . 5 g. de CEMULSOL NP10® de HLB 13,3
   . 5 g. d'eau
   (la viscosité dynamique à un gradient de cisaillement de 1 s⁻¹ de ce mélange tensio-actif/eau est de 253 Pa.s.)
- et d'autre part 90 g. d'huile α,ω-bis(triméthyl)polydiméthylsiloxane ayant une viscosité de 500 Pa.s. (huile 47V500 000)

L'émulsion obtenue présente un extrait sec de 95 %.

L'évolution de la granulométrie moyenne en fonction du temps de malaxage est donnée au tableau 3.

### Exemple 19

On répète l'opération décrite à l'exemple 16 à partir
- d'une part
   . 6,3 g. de CEMULSOL NP10® de HLB 13,3
   . 7,7 g. d'eau
- et d'autre part 86 g. d'une solution à 40 % en poids dans le décaméthylcyclopentasiloxane (D5) de Gomme FB® (gomme α,ω-bis(triméthyl)polydiméthylsiloxane ayant une consistance de l'ordre de 700 à 1000, commercialisée par RHONE-POULENC) ; cette solution de gomme présente une viscosité de 500 Pa.s..

L'émulsion obtenue présente un extrait sec de 92,3 %.

L'évolution de la granulométrie moyenne en fonction du temps de malaxage est donnée au tableau 3.

**TABLEAU 3**

| **durée de malaxage** (en mn) après introduction de l'huile | **granulométrie moyenne** (en µm) exemple | | | |
|---|---|---|---|---|
| | 16 | 17 | 18 | 19 |
| 15 | 1,21 | 1,21 | 2,77 | 1,38 |
| 30 | | 1,16 | | |
| 45 | 1,10 | 1,07 | 1,65 | 1,26 |
| 75 | 1,09 | 1,05 | 1,53 | 1,11 |

### Exemple 20

Dans un réacteur de 250 cm³, muni d'un système d'agitation à pale raclante, on introduit
. 6,3 g. de CEMULSOL NP10®
. 7,7 g. d'eau

Le mélange est agité à 150 tours/min. pendant 5 minutes.

Il se forme une pâte épaisse à laquelle on ajoute à l'aide d'une pompe poussoire située en fond de réacteur 86 g. de Gomme FB, en 20 minutes environ, sous agitation à environ 350 tours/min.. A la fin de l'introduction de l'huile, le mélange est introduit dans la cuve d'un farinographe BRABENDER® (commercialisé par BRABENDER) équipé de deux hélices de malaxage raclantes et malaxé pendant plusieurs heures. On obtient une émulsion présentant un extrait sec de 92,3 %.

L'évolution de la granulométrie moyenne en fonction du temps de malaxage est donnée au tableau 4.

**TABLEAU 4**

| **durée de malaxage** dans le malaxeur BRABENDER (en heures) | 1 | 3 | 6 | 26 |
|---|---|---|---|---|
| **granulométrie moyenne** ( en micromètre) | 6,42 | 3,97 | 2,93 | 1,99 |

### Exemple 21 (comparatif)

On répète l'opération décrite à l'exemple 17 à partir
- d'une part
   . 3 g. de CEMULSOL NP10
   . 11g. d'eau
   (la viscosité de ce milieu aqueux est de 10 mPa.s. environ)
- et d'autre part 86 g. d'huile 47V100 000

L'émulsion obtenue présente une granulométrie moyenne supérieure à 30 micromètres, avec des gouttelettes millimétriques.

### Exemple 22 (comparatif)

On répète l'opération décrite à l'exemple 16 à partir
- d'une part
   . 3 g. de CEMULSOL NP10
   . 11 g. d'eau
- et d'autre part 86 g. d'huile α,ω-bis(triméthyl)polydiméthylsiloxane ayant une viscosité de 30 Pa.s. (huile 47V30 000)

L'émulsion obtenue présente une granulométrie moyenne supérieure à 10 micromètres, avec des gouttellettes d'environ 1 mm.

### Exemple 23

Dans un malaxeur planétaire à turbine NEULINGER® (commercialisé par Neulinger) de 10 litres, on introduit
. 2.500 g d'huile 48 V 175000
. 250 g de CEMULSOL NP 9

Le milieu est agité pendant 6 minutes à l'aide du système planétaire et à 60 tours/minute et de la turbine à 500 tours/minute.

On introduit sous agitation 82 g d'eau en 5 min environ, ce qui correspond à un rapport pondéral eau/eau+tensio-actif de 0,25, puis on reprend l'opération de malaxage comme précédemment pendant 100 min.

L'évolution de la granulométrie moyenne de l'émulsion en fonction du temps de malaxage est donnée dans le tableau 6.

L'émulsion finale est ensuite diluée avec 1467 g d'eau de façon à ajuster son extrait sec à 62,7 %.

**TABLEAU 6**

| **durée de malaxage** après introduction de l'eau (en minutes) | température (°C) | **granulométrie moyenne** (en nanomètres) |
|---|---|---|
| 10 | 27 | 792 |
| 17 | - | 647 |
| 40 | 43 | 511 |
| 100 | 35 | 438 |

### Exemple 24

Dans un réacteur de 250 cm³, muni d'un système d'agitation à pale raclante, on introduit
. 6,3 g. de CEMULSOL NP10®
. 7,7 g. d'eau

Le mélange est agité à 150 tours/min. pendant 5 minutes.

Il se forme une pâte épaisse à laquelle on ajoute à l'aide d'une pompe poussoire située en fond de réacteur 86 g. de Gomme 761® ( gomme polydiphényldiméthylsiloxane commercialisée par RHONE-POULENC) , en 20 minutes environ, sous agitation à environ 350 tours/min.. A la fin de l'introduction de l'huile, le mélange est introduit dans la cuve d'un farinographe BRABENDER® (commercialisé par BRABENDER) équipé de deux hélices de malaxage raclantes et malaxé pendant 30 min. On obtient une émulsion présentant et une granulométrie de 2,4 µm.

Cette émulsion est ensuite diluée par de l'eau jusqu'à obtenir un extrait sec de 50 %.

### Exemple 25

On introduit 1,42 partie en poids de emulsion d'huile silicone visqueuse de l'exemple 3 dans une formulation de shampoing conditionneur de composition suivante :
. 36,0 parties en poids de sodium laureth sulfate*
. 2,0 parties en poids de lauramide DEA*
. 2,0 parties en poids de glycol distearate*
. 1,50 partie en poids de PEG 6000 distearate*
. 0,35 partie en poids de xanthan gum* RHODICARE D® commercialisé par RHONE-POULENC
. 0,65 partie en poids de hydroxypropyl guar gum* JAGUAR HP60® commercialisé par RHONE-POULENC
. 0,5 partie en poids de conservateur
. 0,25 partie en poids de parfum
. 0,25 partie en poids de polysorbate 20* ALKAMULS® commercialisé par RHONE-POULENC
. une quantité d'eau distillée permettant d'obtenir 100 parties en poids de composition finale (formulation de shampoing+émulsion d'huile visqueuse).
[* nom C.T.F.A.(Cosmetic Toiletry Fragante Association) utilisé dans la profession cosmétique et de l'hygiène corporelle]
On constate que l'introduction de l'émulsion d'huile silicone visqueuse facilite le peignage et le coiffage des cheveux secs ou mouillés, augmente la brillance de la chevelure sèche et améliore le séchage des cheveux mouillés.

### Exemple 26

On introduit 1,16 partie en poids de l'emulsion d'huile silicone de l'exemple 18 dans une formulation de shampoing doux conditionneur de composition suivante :
. 36,0 parties en poids de sodium laureth sulfate*
. 4,0 parties en poids de disodium cocoamphodiacetate* MIRANOL C2M CONC NP® commercialisé par RHONE-POULENC
. 2,0 parties de lauramide DEA*
. 2,0 parties en poids de glycol distearate*
. 0,50 partie en poids de PEG 6000 distearate*
. 0,35 partie en poids de xanthan gum* RHODICARE D® commercialisé par RHONE-POULENC
. 0,65 partie en poids de hydroxypropyl guar gum* JAGUAR HP60® commercialisé par RHONE-POULENC
. 0,5 partie en poids de conservateur
. 0,25 partie en poids de parfum
. 0,25 partie en poids de polysorbate 20* ALKAMULS® commercialisé par RHONE-POULENC
. une quantité d'eau distillée permettant d'obtenir 100 parties en poids de composition finale (formulation de shampoing+émulsion d'huile visqueuse).

On constate que l'introduction de l'émulsion d'huile silicone visqueuse facilite le peignage et le coiffage des cheveux secs ou mouillés et améliore le séchage des cheveux mouillés.

### Exemple 27

On introduit 2,0 parties en poids de l'emulsion de gomme silicone de l'exemple 24 dans une formulation de crème solaire de composition suivante :
. 10,0 parties en poids d'huile minérale MARCOL 52® commercialisé par EXXON .
. 5,0 parties en poids d'huile de jojoba*
. 10,0 parties en poids de palmitate d'isopropyle
. 5,0 parties en poids de propanediol-1,2
. 3 parties en poids de filtre U-V RODIALUX A® commercialisé par RHONE-POULENC
. 3 parties en poids de filtre U-V RODIALUX S® commercialisé par RHONE-POULENC
. 0,5 partie en poids de conservateur
. 15,0 parties en poidsde base émulsionnante TEFOSE 63® commercialisé par GATTEFOSSE
. 0,25 partie en poids de parfum
. 0,25 partie en poids de polysorbate 20* ALKAMULS® commercialisé par RHONE-POULENC
. une quantité d'eau distillée permettant d'obtenir 100 parties en poids de composition finale (formulation de crème+émulsion de gomme).

On constate que l'introduction d'émulsion de gomme silicone augmente la rémanence des filtres U-V sur la peau ; l'activité filtrante de cette crème est maintenue même après un bain prolongé.

### Exemple 28

On introduit 1,16 partie en poids de l'emulsion d'huile silicone de l'exemple 18 dans une formulation de mousse à raser de composition suivante :
. 4,8 parties en poids d'acide stéarique
. 1,2 partie en poids d'acide gras de coprah
. 2,65 parties de triéthanolamine
. 3,0 parties en poids de propylèneglycol
. 4,5 parties en poids de glycérol
. 0,25 partie en poids de parfum
. 0,25 partie en poids de polysorbate 20* ALKAMULS® commercialisé par RHONE-POULENC
. 10,0 parties en poids de propulseur (3,2 bar)
. une quantité d'eau distillée permettant d'obtenir 100 parties en poids de composition finale (formulation de mousse à raser+émulsion d'huile visqueuse).

On constate que l'introduction de l'émulsion d'huile silicone visqueuse facilite la glisse du rasoir sur la peau, diminue l'irritation due au rasoir et confère à la peau un toucher très doux après le rasage.

### Exemple 29

On introduit 1,42 partie en poids de l'emulsion d'huile silicone de l'exemple 3 dans une formulation de mousse de coiffage de composition suivante :
. 1,5 partie en poids de cocamidopropyl betaine* MIRATAINE CB® commercialisé par RHONE-POULENC
. 1,5 partie en poids d'oleamidopropyl betaine* MIRATAINE BET-O-30® commercialisé par RHONE-POULENC
. 4,0 parties en poids de polymethacrylamidopropyl trimonium chloride* POLYCARE 133® commercialisé par RHONE-POULENC
. 1,0 partie d'huile silicone SILBIONE 70646® (dimethicone copolyol) commercialisé par RHONE-POULENC
. 0,5 partie de conservateur
. 0,25 partie en poids de parfum
. 0,25 partie en poids de polysorbate 20* ALKAMULS® commercialisé par RHONE-POULENC
. 10,0 parties en poids de propulseur (3,2 bar)
. une quantité d'eau distillée permettant d'obtenir 100 parties en poids de composition finale (formulation de mousse coiffante+émulsion d'huile visqueuse).

On constate que l'introduction d'émulsion d'huile silicone visqueuse permet d'améliorer le peignage, le coiffage et la brillance des cheveux.

## Revendications

1. Procédé de préparation d'émulsions aqueuses d'huiles et/ou de gommes et/ou de résines silicones **caractérisé**
- **en ce qu'**on malaxe un mélange constitué de
. 100 parties en poids d'une phase silicone (A) de viscosité dynamique, mesurée à 25 °C à l'aide d'un viscosimètre Brookfield selon les indications de la norme AFNOR NFT 76 102 de février 1972, au moins égale à 3 Pa.s., ou de consistance déterminée par mesure à 25 °C de la pénétrabilité à l'aide d'un pénétromètre selon les indications de la norme AFNOR NFT 60 123 ou NFT 66 004, inférieure à 2000, phase silicone comprenant au moins une huile et/ou au moins une gomme et/ou au moins une résine polyorganosiloxanique ;
. 2 à 20 parties en poids d'eau ;
. 3 à 20 parties en poids d'au moins un agent tensio-actif (B) de nature non-ionique ou une combinaison de 0,5 à 10 parties en poids d'au moins un agent tensio-actif (B) de nature non-ionique et de 2,5x10⁻⁴ à 20 parties en poids d'au moins un polymère hydrosoluble épaississant (C) de masse moléculaire supérieure à 10 000 g./mole,
- ledit agent tensio-actif ou mélange d'agents tensio-actifs présentant un HLB d'au moins 10 et les quantités relatives d'eau, de(s) constituant(s) (B) et éventuellement (C) étant telles que la viscosité ou la consistance du mélange eau + agent(s) tensio-actif(s) + polymère(s) hydrosoluble(s) épaississant(s) éventuel(s), ladite viscosité ou consistance étant mesurée selon la méthode normalisée indiquée ci-avant, soit voisine du ou supérieure au dixième de la viscosité ou consistance de la phase silicone (A) ;
- ledit malaxage étant réalisé, en mettant en oeuvre une seule étape, dans un malaxeur muni d'un agitateur, agitateur dont la partie mobile ne tourne pas à plus de 2500 tours/min, avec une vitesse tangentielle en extrêmité de partie mobile ne dépassant pas 20 m/s et avec un rapport vitesse tangentielle en extrêmité de partie mobile/distance entre l'extrêmité de la partie mobile et la paroi du malaxeur qui est inférieur à 50000 s⁻¹, pendant une durée suffisante pour obtenir une émulsion de type "huile dans eau" de granulométrie de l'ordre de 0,1 à 5 micromètres ;
- puis en ce qu'on dilue éventuellement le milieu avec de l'eau en fonction du taux d'extrait sec désiré.

2. Procédé selon la revendication 1 **caractérisé :**
- **en ce qu'**on malaxe un mélange constitué de
. 100 parties en poids d'une phase silicone (A) de viscosité dynamique à 25 °C au moins égale à 30 Pa.s., ou de consistance à 25 °C inférieure à 2000, phase silicone comprenant au moins une huile et/ou au moins une gomme et/ou au moins une résine polyorganosiloxanique ;
. 3 à 15 parties en poids d'eau ;
. 5 à 15 parties en poids d'au moins un agent tensio-actif (B) de nature non-ionique ou une combinaison de 1 à 10 parties en poids d'au moins un agent tensio-actif (B) de nature non-ionique et de 0,001 à 15 parties en poids d'au moins un polymère hydrosoluble épaississant (C) de masse moléculaire supérieure à 100 000 g./mole,
- ledit agent tensio-actif ou mélange d'agents tensio-actifs présentant un HLB d'au moins 10 et les quantités relatives d'eau, de(s) constituant(s) (B) et éventuellement (C) étant telles que la viscosité ou la consistance du mélange eau + agent(s) tensio-actif(s) + polymère(s) hydrosoluble(s) épaississant(s) éventuel(s) soit voisine de ou supérieure à la viscosité ou consistance de la phase silicone (A) ;
- ledit malaxage étant réalisé, en mettant en oeuvre une seule étape, dans un malaxeur muni d'un agitateur, agitateur dont la partie mobile ne tourne pas à plus de 2500 tours/min, avec une vitesse tangentielle en extrêmité de partie mobile ne dépassant pas 20 m/s et avec un rapport vitesse tangentielle en extrémité de partie mobile/distance entre l'extrêmité de la partie mobile et la paroi du malaxeur qui est inférieur à 50000 s⁻¹, pendant une durée suffisante pour obtenir une émulsion de type "huile dans eau" de granulométrie de l'ordre de 0,2 à 3 micromètres ;
- puis en ce qu'on dilue éventuellement le milieu avec de l'eau en fonction du taux d'extrait sec désiré.

3. Procédé selon la revendication 1, **caractérisé en ce que** la phase silicone (A) est choisie parmi les phases consistant en
. une huile et/ou une gomme et/ou une résine polyorganosiloxanique de viscosité au moins égale à 3 Pa.s.,
. un mélange d'huile(s) et/ou de gomme(s) et/ou de résine(s) polyorganosiloxanique(s), mélange de viscosité au moins égale à 3 Pa.s.,
. un mélange d'huile(s) et/ou gomme(s) et/ou résine(s) polyorganosiloxanique(s) et d'au moins un solvant de ladite huile et/ou gomme et/ou résine et/ou d'au moins un silane et/ou d'au moins une charge siliceuse ou non siliceuse, mélange de viscosité au moins égale à 3 Pa.s.

4. Procédé selon la revendication 2, **caractérisé en ce que** la phase silicone (A) est choisie parmi les phases consistant en
. une huile et/ou une gomme et/ou une résine polyorganosiloxanique de viscosité de l'ordre de 30 à 2.500 Pa.s.ou de consistance de l'ordre de 200 à 2.000,
. un mélange d'huile(s) et/ou de gomme(s) et/ou de résine(s) polyorganosiloxanique(s), mélange de viscosité de l'ordre de 30 à 2.500 Pa.s.ou de consistance de l'ordre de 200 à 2.000,
. un mélange d'huile(s) et/ou gomme(s) et/ou résine(s) polyorganosiloxanique(s) et d'au moins un solvant de ladite huile et/ou gomme et/ou résine et/ou d'au moins un silane et/ou d'au moins une charge siliceuse ou non siliceuse, mélange de viscosité de l'ordre de 30 à 2.500 Pa.s ou de consistance de l'ordre de 200 à 2.000.

5. Procédé selon l'une quelconque des revendications 1 à 4, **caractérisé en ce que** les huiles et les gommes polyorganosiloxaniques sont constituées des motifs de formule
R'₃₋ₐ Rₐ Si O_{1/2} et R₂ Si O
formules où
- a est un entier de 0 à 3
- les radicaux R sont identiques ou différents et représentent
. un groupe hydrocarboné aliphatique saturé ou insaturé contenant de 1 à 10 atomes de carbone ;
. un groupe hydrocarboné aromatique contenant de 6 à 13 atomes de carbone ;
. un groupe organique polaire lié au silicium par une liaison Si-C ou Si-O-C ;
. un atome d'hydrogène ;
- les radicaux R' sont identiques ou différents et représentent
. un groupe OH
. un groupe alkoxy ou alcényloxy contenant de 1 à 10 atomes de carbone ;
. un groupe aryloxy contenant de 6 à 13 atomes de carbone ;
. un groupe acyloxy contenant de 1 à 13 atomes de carbone
. un groupe cétiminoxy contenant de 1 à 8 atomes de carbone
. un groupe amino- ou amido-fonctionnel contenant de 1 à 6 atomes de carbone, lié au silicium par une liaison Si-N.

6. Procédé selon la revendication 5, **caractérisé en ce qu'**au moins 80 % des radicaux R desdites huiles représentant un groupe méthyle.

7. Procédé selon l'une quelconque des revendications 1 à 4, **caractérisé en ce que** les résines polyorganosiloxanes sont constituées de motifs de formules
R Si O_{3/2} (motif T) et/ou Si O₂ (motif Q)
associés à des motifs de formule
R'₃₋ₐ Rₐ Si O_{1/2} (motif M) et/ou R₂ Si O (motif D)
formules dans lesquelles a, R et R' ont la définition donnée à la revendication 5.

8. Procédé selon l'une des revendications 3 à 7, **caractérisé en ce que** le silane est un sous-produit de synthèse desdites huiles, gommes ou résines polyorganosiloxaniques présent selon des quantités de l'ordre de 0 à 10 parties en poids pour 100 parties en poids d'huile(s) et/ou de gomme(s) et/ou de résine(s) polyorganosiloxanique(s) ou un agent de réticulation desdites huiles, gommes ou résines, présent selon des quantités de l'ordre de 0,5 à 30 parties en poids pour 100 parties en poids d'huile(s) et/ ou de gomme(s) et/ou de résine(s), ledit silane ayant pour formule (R_{b}) Si (OR')_{4-b}, formule dans laquelle b est un entier de 0 à 4, R et R' ayant la définition donnée à la revendication 5.

9. Procédé selon l'une des revendications 3 à 7, **caractérisé en ce que** ledit silane est un agent d'adhérence de compositions silicones, tel que l'aminopropyltriéthoxysilane, l'aminopropylméthyldiéthoxysilane, le glycidoxy-propyltriméthoxysilane, présent selon des quantités pouvant aller jusqu'à 200 % du poids d'huile(s) et/ou de gomme(s) et/ ou résine(s).

10. Procédé selon l'une des revendications 3 à 9, **caractérisé en ce que** les charges siliceuses ou non siliceuses sont des charges renforçantes ou semi-renforçantes de granulométrie de l'ordre de 0,001 à 300 µm., présentes selon des quantités pouvant aller jusqu'à 300 % du poids d'huile(s) et/ou de gomme(s) et/ou de résine(s)

11. Procédé selon l'une des revendications 1 à 10, **caractérisé en ce que** les polymères épaisissants (C) sont solubles à au moins 50 % dans l'eau et sont choisis parmi les alcools polyvinyliques, les polyéthylènes glycols, les polyvinylpyrrolidones, les polyacrylates de métaux alcalins, les carraghénanes, les alginates, la gomme xanthane, les carboxyméthylcelluloses, les méthylcelluloses, les hydroxypropylcelluloses, les hydroxyéthylcelluloses.

12. Procédé selon l'une des revendications 1 à 11, **caractérisé en ce que** l'opération de mise en émulsion de la phase silicone est réalisée par introduction d'au moins une huile et/ou une gomme et/ou au moins une résine dans un mélange eau + agent(s) tensio-actif(s)+polymère(s) hydrosoluble(s) éventuel(s), puis malaxage à une température de l'ordre de 10 à 50 °C.

13. Procédé selon l'une des revendications 3 à 11, **caractérisé en ce que** l'opération de mise en émulsion de la phase silicone est réalisée par introduction d' au moins une huile et/ou une gomme et/ou au moins une résine +solvant(s) éventuel(s)+ silane(s) éventuel(s) dans un mélange eau + agent(s) tensio-actif(s)+polymère hydrosoluble éventuel , la ou les charge(s) eventuelle(s) étant présente(s) dans le mélange aqueux et/ou introduite(s) dans ledit mélange, puis malaxage à une température de l'ordre de 10 à 50 °C.

14. Procédé selon l'une des revendications 3 à 11, **caractérisé en ce que** l'opération de mise en émulsion de la phase silicone est réalisée par introduction de l'eau dans un mélange huile(s) et/ou gomme(s) et/ou résine(s) présente(s) en totalité ou partiellement + solvant(s) éventuel(s) + silane(s) éventuel(s) + charge(s) éventuelle(s)+ agent(s) tensio-actif(s) + polymère hydrosoluble éventuel, puis malaxage à une température de l'ordre de 10 à 50 °C, la quantité éventuellement restante d'huile(s) et/ou gomme(s) et/ou résine(s) étant introduite dans le milieu après la formation de l'émulsion "huile dans eau" tout en maintenant le malaxage.

15. Procédé selon l'une des revendications 1 à 14, **caractérisé en ce que** l'opération de malaxage est réalisée dans un malaxeur muni d'un agitateur, agitateur dont la partie mobile ne tourne pas à plus de 1500 tours/mn avec une vitesse tangentielle en extrêmité de partie mobile ne dépassant pas 5m/s et avec un rapport vitesse tangentielle en extrémité de partie mobile / distance entre l'extrémité de la partie mobile et la paroi du malaxeur qui est inférieur à 10000s⁻¹.

16. Procédé selon l'une des revendications 1 à 14, **caractérisé en ce que** l'opération de malaxage est réalisée dans un malaxeur muni d'un agitateur, agitateur dont la partie mobile ne tourne pas à plus de 500 tours/mn avec une vitesse tangentielle en extrêmité de partie mobile ne dépassant pas 2,5m/s, et avec un rapport vitesse tangentielle en extrémité de partie mobile / distance entre l'extrémité de la partie mobile et la paroi du malaxeur qui est inférieur à 2500s⁻¹.

17. Procédé selon l'une des revendications 1 à 16, **caractérisé en ce que** l'opération de malaxage est réalisée dans une extrudeuse à vis simple ou multiple(s), un malaxeur planétaire, un malaxeur à crochet, un disperseur lent, un malaxeur statique, un malaxeur à pale, à hélice, à bras, à ancre.

18. Utilisation des émulsions aqueuses d'huiles et/ou de gommes et/ou de résines silicones obtenues selon le procédé faisant l'objet de l'une quelconque des revendications 1 à 17 pour la préparation de compositions cosmétiques.

## Patentansprüche

1. Verfahren zur Herstellung wässeriger Emulsionen von Siliconölen und/oder Silicongummis und/oder Siliconharzen, **dadurch gekennzeichnet, dass**
- ein Gemisch verknetet wird, das sich aus folgenden Bestandteilen zusammensetzt:
· 100 Gewichtsteilen einer Siliconphase (A), die bei 25 °C eine mit Hilfe eines Brookfield-Viskosimeters entsprechend der Norm AFNOR NFT 76 102 vom
Februar 1972 bestimmte dynamische Viskosität von mindestens 3 Pa.s. aufweist oder die bei 25 °C eine mit Hilfe eines Penetrationsmessers entsprechend der Norm AFNOR NFT 60 123 oder NFT 66 004 durch Messung der Penetration bestimmte Konsistenz kleiner 2000 aufweist, wobei diese Siliconphase mindestens ein Polyorganosiloxanöl und/oder mindestens ein Polyorganosiloxangummi und/oder mindestens ein Polyorganosiloxanharz
enthält,
· 2 bis 20 Gewichtsteilen Wasser
und
· 3 bis 20 Gewichtsteilen mindestens eines nichtionischen grenzflächenaktiven Mittels (B) oder 0,5 bis 10 Gewichtsteilen mindestens eines nichtionischen grenzflächenaktiven Mittels (B) in Kombination mit 2,5 · 10⁻⁴ bis 20 Gewichtsteilen mindestens eines wasserlöslichen polymeren Verdickungsmittels (C) einer Molmasse größer 10 000 g/Mol, wobei dieses grenzflächenaktive Mittel beziehungsweise dieses Gemisch grenzflächenaktiver Mittel einen HLB-Wert (Verhältnis Hydrophilie/Lipophilie) von mindestens 10 aufweist und das Wasser und der beziehungsweise die Bestandteile (B) sowie gegebenenfalls (C) in solchen relativen Mengen vorliegen, dass die Viskosität beziehungsweise die Konsistenz des Gemischs *Wasser + grenzflächenaktives Mittel (beziehungsweise grenzfächenaktive Mittel) + gegebenenfalls vorhandenes wasserlösliches polymeres Verdickungsmittel (beziehungsweise wasserlösliche polymere Verdickungsmittel)*, die entsprechend der oben angegebenen genormten Methode ermittelt wird, im Bereich eines Zehntels der Viskosität beziehungsweise der Konsistenz der Siliconphase (A) oder darüber liegt,
wobei dieses Verkneten in einem einzigen Arbeitsschritt in einer mit einem Rührwerk ausgestatteten Mischapparatur, in der sich der bewegliche Teil dieses Rührwerks mit maximal 2500 Umdrehungen/min dreht, die Tangentialgeschwindigkeit am äußeren Ende des beweglichen Teils 20 m/s nicht überschreitet und das Verhältnis *Tangentialgeschwindigkeit am äußeren Ende des* *beweglichen Teils*/*Entfernung zwischen dem äußeren Ende des beweglichen Teils und der Mischerwand* kleiner 50 000 s⁻¹ ist, und über einen Zeitraum durchgeführt wird, der ausreicht, um eine "Öl-in-Wasser"-Emulsion einer mittleren Teilchengröße in der Größenordnung von 0,1 bis 5 µm zu erhalten;
und
- das Medium in Abhängigkeit vom gewünschten Gehalt an Trockensubstanz gegebenenfalls mit Wasser verdünnt wird.

2. Verfahren nach Anspruch 1, **dadurch gekennzeichnet, dass**
- ein Gemisch verknetet wird, das sich aus folgenden Bestandteilen zusammensetzt:
· 100 Gewichtsteilen einer Siliconphase (A), die bei 25 °C eine dynamische Viskosität von mindestens 30 Pa.s. aufweist oder die bei 25 °C eine Konsistenz kleiner 2000 aufweist, wobei diese Siliconphase mindestens ein Polyorganosiloxanöl und/oder mindestens ein Polyorganosiloxangummi und/oder mindestens ein Polyorganosiloxanharz enthält,
· 3 bis 15 Gewichtsteilen Wasser
und
· 5 bis 15 Gewichtsteilen mindestens eines nichtionischen grenzflächenaktiven Mittels (B) oder 1 bis 10 Gewichtsteilen mindestens eines nichtionischen grenzflächenaktiven Mittels (B) in Kombination mit 0,001 bis 15 Gewichtsteilen mindestens eines wasserlöslichen polymeren Verdickungsmittels (C) einer Molmasse größer 100 000 g/Mol, wobei dieses grenzflächenaktive Mittel beziehungsweise dieses Gemisch grenzflächenaktiver Mittel einen HLB-Wert von mindestens 10 aufweist und das Wasser und der beziehungsweise die Bestandteile (B) sowie gegebenenfalls (C) in solchen relativen Mengen vorliegen, dass die Viskosität beziehungsweise die Konsistenz des Gemischs *Wasser + grenzflächenaktives Mittel (beziehungsweise grenzfächenaktive Mittel) + gegebenenfalls vorhandenes wasserlösliches polymeres Verdickungsmittel (beziehungsweise wasserlösliche polymere Verdickungsmittel) im Bereich der Viskosität beziehungsweise der Konsistenz* der Siliconphase (A) oder darüber liegt,
wobei dieses Verkneten in einem einzigen Arbeitsschritt in einer mit einem Rührwerk ausgestatteten Mischapparatur, in der sich der bewegliche Teil dieses Rührwerks mit maximal 2500 Umdrehungen/min dreht, die Tangentialgeschwindigkeit am äußeren Ende des beweglichen Teils 20 m/s nicht überschreitet und das Verhältnis *Tangentialgeschwindigkeit am äußeren Ende* *des beweglichen Teils*/*Entfernung zwischen dem äußeren Ende des beweglichen Teils und der Mischerwand* kleiner 50 000 s⁻¹ ist, und über einen Zeitraum durchgeführt wird, der ausreicht, um eine "Öl-in-Wasser"-Emulsion einer mittleren Teilchengröße in der Großenordnung von 0,2 bis 3 µm zu erhalten,
und
- das Medium in Abhängigkeit vom gewünschten Gehalt an Trockensubstanz gegebenenfalls mit Wasser verdünnt wird.

3. Verfahren nach Anspruch 1, **dadurch gekennzeichnet, dass** die Siliconphase (A) unter den Phasen ausgewählt ist, die bestehen aus:
- einem Polyorganosiloxanöl und/oder einem Polyorganosiloxangummi und/oder einem Polyorganosiloxanharz, wobei diese Phase eine Viskosität von mindestens 3 Pa.s, aufweist,
- einem Gemisch aus einem oder mehreren Polyorganosiloxanölen und/oder einem oder mehreren Polyorganosiloxangummis und/oder einem oder mehreren Polyorganosiloxanharzen, wobei dieses Gemisch eine Viskosität von mindestens 3 Pa.s. aufweist,
oder
- einem Gemisch aus einem oder mehreren Polyorganosiloxanölen und/oder einem oder mehreren Polyorganosiloxangummis und/oder einem oder mehreren Polyorganosiloxanharzen und mindestens einem Lösungsmittel dieses Öls und/oder Gummis und/oder Harzes und/oder mindestens einem Silan und/oder mindestens einem kieselsäurehaltigen oder nicht kieselsäurehaltigen Füllstoff, wobei dieses Gemisch eine Viskosität von mindestens 3 Pa.s. aufweist.

4. Verfahren nach Anspruch 2, **dadurch gekennzeichnet, daß** die Siliconphase (A) unter den Phasen ausgewählt ist, die bestehen aus:
- einem Polyorganosiloxanöl und/oder einem Polyorganosiloxangummi und/oder einem Polyorganosiloxanharz, wobei diese Phase eine Viskosität in der Größenordnung von 30 bis 2500 Pa.s. beziehungsweise eine Konsistenz in der Größenordnung von 200 bis 2000 aufweist,
- einem Gemisch aus einem oder mehreren Polyorganosiloxanölen und/oder einem oder mehreren Polyorganosiloxangummis und/oder einem oder mehreren Polyorganosiloxanharzen, wobei dieses Gemisch eine Viskosität in der Größenordnung von 30 bis 2500 Pa.s. beziehungsweise eine Konsistenz in der Größenordnung von 200 bis 2000 aufweist,
oder
- einem Gemisch aus einem oder mehreren Polyorganosiloxanölen und/oder einem oder mehreren Polyorganosiloxangummis und/oder einem oder mehreren Polyorganosiloxanharzen und mindestens einem Lösungsmittel dieses Öls und/oder Gummis und/oder Harzes und/oder mindestens einem Silan und/oder mindestens einem kieselsäurehaltigen oder nicht kieselsäurehaltigen Füllstoff, wobei dieses Gemisch eine Viskosität in der Größenordnung von 30 bis 2500 Pa.s. beziehungsweise eine Konsistenz in der Größenordnung von 200 bis 2000 aufweist.

5. Verfahren nach einem der Ansprüche 1 bis 4, **dadurch gekennzeichnet, dass** die Polyorganosiloxanöle und -gummis aus Einheiten entsprechend den folgenden Formeln aufgebaut sind:
R'₃₋ₐRₐSiO_{1/2} und R₂SiO
in denen:
- a eine ganze Zahl von 0 bis 3 ist,
- die Reste R, die gleich oder verschieden sein können,
• eine gesättigte oder ungesättigte aliphatische Kohlenwasserstoffgruppe mit 1 bis 10 Kohlenstoffatomen,
• eine aromatische Kohlenwasserstoffgruppe mit 6 bis 13 Kohlenstoffatomen,
• eine polare organische Gruppe, die über eine Si-C- oder eine Si-O-C - Bindung an das Siliciumatom gebunden ist, oder
• ein Wasserstoffatom darstellen,
und
- die Reste R', die gleich oder verschieden sein können,
• eine OH-Gruppe,
• eine Alkoxy- oder eine Alkenyloxygruppe mit 1 bis 10 Kohlenstoffatomen,
• eine Aryloxygruppe mit 6 bis 13 Kohlenstoffatomen,
• eine Acyloxygruppe mit 1 bis 13 Kohlenstoffatomen,
• eine Ketiminoxy-Gruppe mit 1 bis 8 Kohlenstoffatomen
oder
• eine amino-oder amidofunktionelle Gruppe mit 1 bis 6 Kohlenstoffatomen darstellen, die über eine Si-N-Bindung an das Siliciumatom gebunden ist.

6. Verfahren nach Anspruch 5, **dadurch gekennzeichnet, dass** mindestens 80 % der Reste R der besagten Öle eine Methylguppe darstellen.

7. Verfahren nach einem der Ansprüche 1 bis 4, **dadurch gekennzeichnet, dass** die Polyorganosiloxanharze aus Einheiten entsprechend den folgenden Formeln aufgebaut sind:
RSiO_{3/2} (T-Einheiten) und/oder SiO₂ (Q-Einheiten)
in Verbindung mit Einheiten entsprechend den folgenden Formeln:
R'₃₋ₐRₐSiO_{1/2} (M-Einheiten) und/oder R₂SiO (D-Einheiten),
wobei a, R und R' in diesen Formeln jeweils die in Anspruch 5 angegebene Bedeutung haben.

8. Verfahren nach einem der Ansprüche 3 bis 7, **dadurch gekennzeichnet, dass** das Silan ein Nebenprodukt der Synthese der besagten Polyorganosiloxanöle, -gummis oder - harze, das in einer Menge in der Größenordnung von 0 bis 10 Gewichtsteilen pro 100 Gewichtsteilen des beziehungsweise der Polyorganosiloxanöle und/oder des beziehungsweise der Polyorganosiloxangummis und/oder des beziehungsweise der Polyorganosiloxanharze vorliegt, oder ein Vernetzungsmittel dieser Öle, Gummis oder Harze ist, das in einer Menge in der Größenordnung von 0,5 bis 30 Gewichtsteilen pro 100 Gewichtsteilen des beziehungsweise der Öle und/oder des beziehungsweise der Gummis und/oder des beziehungsweise der Harze vorliegt, wobei dieses Silan der Formel (R_{b})Si(OR')_{4-b} entspricht, in der b eine ganze Zahl von 0 bis 4 darstellt und R und R' jeweils die in Anspruch 5 angegebene Bedeutung haben.

9. Verfahren nach einem der Ansprüche 3 bis 7, **dadurch gekennzeichnet, dass** das besagte Silan ein Haftungsmittel für Siliconzusammensetzungen, wie Aminopropyltriethoxysilan, Aminopropylmethyldiethoxysilan oder Glycidyloxypropyltrimethoxysilan, ist, das in einer Menge vorliegt, die bis zu 200 Gew.-% der Menge an Öl (beziehungsweise Ölen) und/oder an Gummi (beziehungsweise Gummis) und/oder an Harz (beziehungsweise Harzen) betragen kann.

10. Verfahren nach einem der Ansprüche 3 bis 9, **dadurch gekennzeichnet, dass** die kieselsäurehaltigen oder nicht kieselsäurehaltigen Füllstoffe verstärkende oder halbverstärkende Füllstoffe einer mittleren Teilchengröße in der Größenordnung von 0,001 bis 300 µm sind, die in einer Menge vorliegen, die bis zu 300 Gew.-% der Menge an Öl (beziehungsweise Ölen) und/oder an Gummi (beziehungsweise Gummis) und/oder an Harz (beziehungsweise Harzen) betragen kann.

11. Verfahren nach einem der Ansprüche 1 bis 10, **dadurch gekennzeichnet, dass** die polymeren Verdickungsmittel (C) zu mindestens 50 % wasserlöslich sind und unter den Polyvinylalkoholen, den Polyethylenglykolen, den Polyvinylpyrrolidonen, den Alkalimetallsalzen der Polyacrylsäure, den Carrageenanen, den Alginaten, dem XanthanGummi, den Carboxymethylcellulosen, den Methylcellulosen, den Hydroxypropylcellulosen und den Hydroxyethylcellulosen ausgewählt sind.

12. Verfahren nach einem der Ansprüche 1 bis 11, **dadurch gekennzeichnet, dass** der Arbeitsschritt des Emulgierens der Siliconphase durchgeführt wird, indem mindestens ein Öl und/oder ein Gummi und/oder mindestens ein Harz zu einem Gemisch *Wasser + grenzflächenaktives Mittel (beziehungsweise grenzflächenaktive Mittel) + gegebenenfalls wasserlösliches Polymer (beziehungsweise wasserlösliche Polymere)* gegeben und dieses Gemisch anschließend bei einer Temperatur in der Größenordnung von 10 bis 50 °C verknetet wird.

13. Verfahren nach einem der Ansprüche 3 bis 11, **dadurch gekennzeichnet, dass** der Arbeitsschritt des Emulgierens der Siliconphase durchgeführt wird, indem mindestens ein Öl und/oder ein Gummi und/oder mindestens ein Harz + gegebenenfalls ein Lösungsmittel (beziehungsweise mehrere Lösungsmittel) + gegebenenfalls ein Silan (beziehungsweise mehrere Silane) zu einem Gemisch *Wasser + grenzflächenaktives Mittel (beziehungsweise grenzflächenaktive Mittel) + gegebenenfalls wasserlösliches Polymer (beziehungsweise wasserlösliche Polymere)* gegeben wird - wobei der beziehungsweise die Füllstoffe gegebenenfalls ebenfalls in diesem wässerigen Gemisch enthalten sind oder aber diesem beigegeben werden - und dieses Gemisch anschließend bei einer Temperatur in der Größenordnung von 10 bis 50 °C verknetet wird.

14. Verfahren nach einem der Ansprüche 3 bis 11, **dadurch gekennzeichnet, dass** der Arbeitsschritt des Emulgierens der Siliconphase durchgeführt wird, indem Wasser zu einem Gemisch *Öl (beziehungsweise Öle) und*/*oder Gummi (beziehungsweise Gummis) und*/*oder Harz (beziehungsweise Harze) (wobei diese Ole, Gummis oder Harze in ihrer Gesamtmenge oder aber in Teilmengen m diesem Gemisch vorliegen) + gegebenenfalls ein oder mehrere Lösungsmittel + gegebenenfalls ein oder mehrere Silane + gegebenenfalls ein oder mehrere Füllstoffe + gegebenenfalls ein oder mehrere grenzflächenaktive Mittel + gegebenenfalls wasserlösliches Polymer* gegeben und dieses Gemisch anschließend bei einer Temperatur in der Größenordnung von 10 bis 50 °C verknetet wird, wobei die gegebenenfalls verbleibende Restmenge an Öl (beziehungsweise Ölen) und/oder Gummi (beziehungsweise Gummis) und/oder Harz (beziehungsweise Harzen) unter Fortführung des Knetvorgangs dem Medium beigegeben wird, wenn sich die "Öl in Wasser"-Emulsion gebildet hat.

15. Verfahren nach einem der Ansprüche 1 bis 14, **dadurch gekennzeichnet, dass** der Arbeitsschritt des Verknetens in einer mit einem Rührwerk ausgestatteten Mischapparatur durchgeführt wird, wobei sich der bewegliche Teil des Rührwerks mit einer Geschwindigkeit von maximal 1500 U/min dreht, die Tangentialgeschwindigkeit am äußeren Ende des beweglichen Teils 5 m/s nicht überschreitet und das Verhältnis *Tangentialgeschwindigkeit am äußeren Ende des beweglichen Teils* / *Entfernung zwischen dem äußeren Ende des beweglichen Teils und der Mischerwand* kleiner 10 000 s⁻¹ ist.

16. Verfahren nach einem der Ansprüche 1 bis 14, **dadurch gekennzeichnet, dass** der Arbeitsschritt des Verknetens in einer mit einem Rührwerk ausgestatteten Mischapparatur durchgeführt wird, wobei sich der bewegliche Teil des Rührwerks mit einer Geschwindigkeit von maximal 500 U/min dreht, die Tangentialgeschwindigkeit am äußeren Ende des beweglichen Teils 2,5 m/s nicht überschreitet und das Verhältnis *Tangentialgeschwindigkeit am äußeren Ende des beweglichen Teils* / *Entfernung zwischen dem äußeren Ende des beweglichen Teils und der Mischerwand* kleiner 2 500 s⁻¹ ist.

17. Verfahren nach einem der Ansprüche 1 bis 16, **dadurch gekennzeichnet, dass** der Arbeitsschritt des Verknetens in einem Ein- oder Mehrschneckenextruder, einem Planetenmischer, einem Mischer mit Knethaken, einer langsamen Dispergiermaschine, einem statischen Mischer oder einem Schaufel-, Schrauben-, Blatt- oder Ankerrührer durchgeführt wird.

18. Verwendung der wässerigen Emulsionen von Siliconölen und/oder von Silicongummis und/oder von Siliconharzen, die nach dem Verfahren, welches Gegenstand eines der Anprüche von 1 bis 17 ist, erhalten werden, zur Herstellung von kosmetischen Zusammensetzungen.

## Claims

1. Process for the preparation of aqueous emulsions of silicone oils and/or gums and/or resins, **characterized**
- **in that** a mixture comprising
• 100 parts by weight of a silicone phase (A) with a dynamic viscosity, measured at 25°C with the aid of a Brookfield viscometer according to the instructions of the AFNOR standard NFT 76 102 of February 1972, at least equal to 3 Pa·s, or with a consistency, determined by measuring, at 25°C, the penetrability with the aid of a penetrometer according to the instructions of the AFNOR standard NFT 60 123 or NFT 66 004, of less than 2000, the silicone phase comprising at least one polyorganosiloxane oil and/or gum and/or resin;
• 2 to 20 parts by weight of water;
• 3 to 20 parts by weight of at least one surface-active agent (B) of nonionic nature or a combination of 0.5 to 10 parts by weight of at least one surface-active agent (B) of nonionic nature and of 2.5 × 10⁻⁴ to 20 parts by weight of at least one water-soluble thickening polymer (C) with a molecular mass greater than 10,000 g/mol,
is kneaded,
- the said surface-active agent or mixture of surface-active agents having an HLB value of at least 10 and the relative amounts of water, constituent(s) (B) and optionally (C) being such that the viscosity or the consistency of the water + surface-active agent(s) + optional water-soluble thickening polymer(s) mixture, the said viscosity or consistency being measured according to the standardized method indicated above, is in the region of or greater than one-tenth of the viscosity or consistency of the silicone phase (A);
- the said kneading being carried out by using a single stage in a mixer equipped with a stirrer, in which stirrer the moving part does not rotate at more than 2500 revolutions/min with a tangential speed at the end of the moving part not exceeding 20 m/s and with a tangential speed at the end of the moving part/distance between the end of the moving part and the wall of the mixer ratio which is less than 50,000 s⁻¹, for a period of time which is sufficient to produce an emulsion of "oil-in-water" type with a particle size of the order of 0.1 to 5 micrometres;
- and then in that the medium is optionally diluted with water according to the desired solids content.

2. Process according to claim 1, **characterized**
- **in that** a mixture comprising
• 100 parts by weight of a silicone phase (A) with a dynamic viscosity at 25°C at least equal to 30 Pa·s, or with a consistency at 25°C of less than 2000, the silicone phase comprising at least one polyorganosiloxane oil and/or gum and/or resin;
• 3 to 15 parts by weight of water;
• 5 to 15 parts by weight of at least one surface-active agent (B) of nonionic nature or a combination of 1 to 10 parts by weight of at least one surface-active agent (B) of nonionic nature and of 0.001 to 15 parts by weight of at least one water-soluble thickening polymer (C) with a molecular mass greater than 100,000 g/mol,
is kneaded,
- the said surface-active agent or mixture of surface-active agents having an HLB value of at least 10 and the relative amounts of water, constituent(s) (B) and optionally (C) being such that the viscosity or the consistency of the water + surface-active agent(s) + optional water-soluble thickening polymer(s) mixture is in the region of or greater than the viscosity or consistency of the silicone phase (A);
- the said kneading being carried out by using a single stage in a mixer equipped with a stirrer, in which stirrer the moving part does not rotate at more than 2500 revolutions/min with a tangential speed at the end of the moving part not exceeding 20 m/s and with a tangential speed at the end of the moving part/distance between the end of the moving part and the wall of the mixer ratio which is less than 50,000 s⁻¹, for a period of time which is sufficient to produce an emulsion of "oil-in-water" type with a particle size of the order of 0.2 to 3 micrometres;
- and then in that the medium is optionally diluted with water according to the desired solids content.

3. Process according to claim 1, **characterized in that** the silicone phase (A) is chosen from the phases consisting of
• a polyorganosiloxane oil and/or gum and/or resin with a viscosity at least equal to 3 Pa·s,
• a mixture of polyorganosiloxane oil(s) and/or gum(s) and/or resin(s), which mixture has a viscosity at least equal to 3 Pa·s,
• a mixture of polyorganosiloxane oil(s) and/or gum(s) and/or resin(s) and of at least one solvent of the said oil and/or gum and/or resin and/or of at least one silane and/or of at least one siliceous or non-siliceous filler, which mixture has a viscosity at least equal to 3 Pa·s.

4. Process according to claim 2, **characterized in that** the silicone phase (A) is chosen from the phases consisting of
• a polyorganosiloxane oil and/or gum and/or resin with a viscosity of the order of 30 to 2500 Pa·s, or with a consistency of the order of 200 to 2000,
• a mixture of polyorganosiloxane oil(s) and/or gum(s) and/or resin(s), which mixture has a viscosity of the order of 30 to 2500 Pa·s or a consistency of the order of 200 to 2000,
• a mixture of polyorganosiloxane oil(s) and/or gum(s) and/or resin(s) and of at least one solvent of the said oil and/or gum and/or resin and/or of at least one silane and/or of at least one siliceous or non-siliceous filler, which mixture has a viscosity of the order of 30 to 2500 Pa·s or a consistency of the order of 200 to 2000.

5. Process according to any one of claims 1 to 4, **characterized in that** the polyorganosiloxane oils and gums comprise the units of formula
R'₃₋ₐRₐSiO_{1/2} and R₂SiO
in which formulae
- a is an integer from 0 to 3
- the R radicals are identical or different and represent
• a saturated or unsaturated aliphatic hydrocarbon group containing from 1 to 10 carbon atoms;
• an aromatic hydrocarbon group containing from 6 to 13 carbon atoms;
• a polar organic group bonded to the silicon by an Si-C or Si-O-C bond;
• a hydrogen atom;
- the R' radicals are identical or different and represent
• an OH group
• an alkoxy or alkenyloxy group containing from 1 to 10 carbon atoms;
• an aryloxy group containing from 6 to 13 carbon atoms;
• an acyloxy group containing from 1 to 13 carbon atoms
• a ketiminoxy group containing from 1 to 8 carbon atoms
• an amino- or amidofunctional group containing from 1 to 6 carbon atoms, bonded to the silicon by an Si-N bond.

6. Process according to claim 5, **characterized in that** at least 80% of the R radicals of the said oils represent a methyl group.

7. Process according to any one of claims 1 to 4, **characterized in that** the polyorganosiloxane resins comprise units of formulae
RSiO_{3/2} (T unit) and/or SiO₂ (Q unit)
in combination with units of formula
R'₃₋ₐRₐSiO_{1/2} (M unit) and/or R₂SiO (D unit)
in which formulae a, R and R' have the definition given in claim 5.

8. Process according to one of claims 3 to 7, **characterized in that** the silane is a by-product from the synthesis of the said polyorganosiloxane oils, gums or resins present in amounts of the order of 0 to 10 parts by weight per 100 parts by weight of polyorganosiloxane oil(s) and/or gum(s) and/or resin(s) or a crosslinking agent of the said oils, gums or resins present in amounts of the order of 0.5 to 30 parts by weight per 100 parts by weight of oil(s) and/or gum(s) and/or resin(s), the said silane having the formula (R_{b})Si(OR')_{4-b}, in which formula b is an integer from 0 to 4, R and R' having the definition given in claim 5.

9. Process according to one of claims 3 to 7, **characterized in that** the said silane is an agent for adhesion of silicone compositions, such as aminopropyltriethoxysilane, aminopropylmethyldiethoxysilane or glycidoxypropyltrimethoxysilane, present in amounts which can range up to 200% of the weight of oil(s) and/or gum(s) and/or resin(s).

10. Process according to one of Claims 3 to 9, **characterized in that** the siliceous or non-siliceous fillers are reinforcing or semi-reinforcing fillers with a particle size of the order of 0.001 to 300 µm present in amounts which can range up to 300% of the weight of oil(s) and/or gum(s) and/or resin(s).

11. Process according to one of claims 1 to 10, **characterized in that** the thickening polymers (C) are soluble to at least 50% in water and are chosen from poly(vinyl alcohol)s, poly(ethylene glycol)s, polyvinylpyrrolidones, alkali metal polyacrylates, carrageenans, alginates, xanthan gum, carboxymethyl celluloses, methyl celluloses, hydroxypropyl celluloses or hydroxyethyl celluloses.

12. Process according to one of claims 1 to 11, **characterized in that** the operation of emulsifying the silicone phase is carried out by introduction of at least one oil and/or gum and/or resin into a water + surface-active agent(s) + optional water-soluble polymer(s) mixture and then kneading at a temperature of the order of 10 to 50°C.

13. Process according to one of claims 3 to 11, **characterized in that** the operation of emulsifying the silicone phase is carried out by introduction of at least one oil and/or gum and/or resin + optional solvent(s) + optional silane(s) into a water + surface-active agent(s) + optional water-soluble polymer mixture, the optional filler(s) being present in the aqueous mixture and/or introduced into the said mixture, and then kneading at a temperature of the order of 10 to 50°C.

14. Process according to one of claims 3 to 11, **characterized in that** the operation of emulsifying the silicone phase is carried out by introduction of water into an oil(s) and/or gum(s) and/or resin(s) mixture, present entirely or partially, + optional solvent(s) + optional silane(s) + optional filler(s) + surface-active agent(s) + optional water-soluble polymer, and then kneading at a temperature of the order of 10 to 50°C, the optionally remaining amount of oil(s) and/or gum(s) and/or resin(s) being introduced into the medium after the formation of the "oil-in-water" emulsion while continuing to knead.

15. Process according to one of claims 1 to 14, **characterized in that** the kneading operation is carried out in a mixer equipped with a stirrer, in which stirrer the moving part does not rotate at more than 1500 revolutions/min with a tangential speed at the end of the moving part not exceeding 5 m/s and with a tangential speed at the end of the moving part/distance between the end of the moving part and the wall of the mixer ratio which is less than 10,000 s⁻¹.

16. Process according to one of claims 1 to 14, **characterized in that** the kneading operation is carried out in a mixer equipped with a stirrer, in which stirrer the moving part does not rotate at more than 500 revolutions/min with a tangential speed at the end of the moving part not exceeding 2.5 m/s and with a tangential speed at the end of the moving part/distance between the end of the moving part and the wall of the mixer ratio which is less than 2500 s⁻¹.

17. Process according to one of claims 1 to 16, **characterized in that** the kneading operation is carried out in a single- or multiple-screw extruder, a planetary mixer, a hook mixer, a slow disperser, a static mixer or a paddle, propeller, arm or anchor mixer.

18. Use of the aqueous emulsions of silicone oils and/or gums and/or resins obtained according to the process forming the subject of any one of claims 1 to 17 for the preparation of cosmetic compositions.
